# EUROPEAN PATENT SPECIFICATION

(11) **EP 4 048 673 B1**
(45) Date of publication and mention of the grant of the patent: **06.12.2023**
(21) Application number: 20789969.1
(22) Date of filing: 12.10.2020
(51) Int. Cl.: C07D 487/04

(54) **PROCESS FOR THE PRODUCTION OF CYCLIC GUANIDINE DERIVATES**
VERFAHREN ZUR HERSTELLUNG VON CYCLISCHEN GUANIDINDERIVATEN
PROCÉDÉ DE PRODUCTION DE DÉRIVÉS DE GUANIDINE CYCLIQUES

(30) Priority: 22.10.2019 EP 19204505
(43) Date of publication of application: 31.08.2022
(73) Proprietor: BASF SE, 67056 Ludwigshafen am Rhein (DE)
(72) Inventor: ERNST, Martin, 67056 Ludwigshafen (DE); HUBER, Tatjana, 67056 Ludwigshafen (DE); LANGLOTZ, Bjoern, 67056 Ludwigshafen (DE)
(74) Representative: BASF IP Association
(86) International application number: PCT/EP2020/078610
(87) International publication number: WO 2021/078563

(56) References cited:
- WO-A1-2011/079041
- WO-A1-2019/092319
- SCHMIDTCHEN F P: "Synthese symmetrisch substituierter bicyclischer Guanidine", CHEMISCHE BERICHTE, VCH, DE, vol. 113, no. 6, 1 January 1980 (1980-01-01), pages 2175-2182, XP002542381, ISSN: 0009-2940, DOI: 10.1002/CBER.19801130612

## Description

The present invention relates to a process for the production of cyclic guanidine derivates of formula I or mixtures of them 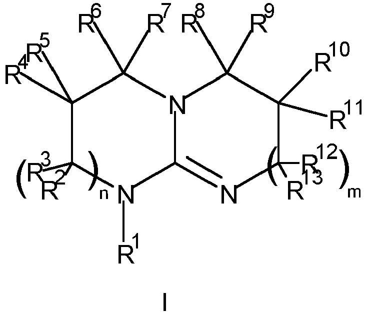 by reacting a triamine in the presence of a C₁-source and a solid material in the gas or liquid phase under inert atmosphere.

Cyclic guanidine derivates of formula I with R¹ to R¹³ independently selected from the group of H and C₁ to C₄-alkyl groups are valuable and active chemical products. They are used as catalysts for a variety of different chemical reactions. Published methods for synthesizing bicyclic guanidines are often complicated, often involve the use of a multiple step synthesis process, and/or require the use of prohibitively expensive starting materials which may be hazardous in a variety of ways as mentioned in US-A1 2009/0281314.

Furthermore, cyclic guanidine derivatives, such as 7-methyl-1,5,7-triazabicyclo[5.5.0]dec-5-ene (Me-TBD), which are treated with organic acids to give a cationic 1,5,7-triazabicyclo[4.4.0]dec-5-enium moiety and an anion can be used as ionic liquids. These ionic liquids can be used as solvents in a process for making cellulose fibers or films as described in WO 2018/138416. They show good hydrothermal stability and are able to dissolve the wood pulp. The resulting solutions are used for spinning textile fibers.

WO 2019/030197describes a method for producing cyclic urea units by reacting triamines with CO₂. The use of triamines with CO₂ in the present of a solid material or a strong organic base in the gas or liquid phase to create cyclic guanidine derivates is not disclosed.

WO 2019/092319 A1 discloses a process for the preparation of cyclic guanidine derivatives.

US-A1 2009/0281314 describes a method for producing bicyclic guanidines by heating the reaction mixture comprising urea and a dehydrating agent like hexamethyldisilazane (HMDS), tetraethoxysilane (TEOS), disilazane, alkoxy substituted silane or combinations thereof to a temperature ≥90°C. US-A1 2009/0281314 does not disclose a method for the production of bicyclic guanidines in the gas or liquid phase in the presence of a solid material. A method to produce the cyclic guanidine derivatives in only one process step is also not disclosed in US-A1 2009/0281314.

Therefore, it is an object of the present invention to provide a process for the production of cyclic guanidine derivatives of formula I that is environmentally benign, generates less waste than the processes described above and with a maximum of three process steps, preferably in one process step.

Unexpectedly, it was found that this can be accomplished with a process for the production of cyclic guanidine derivates of formula I or mixtures of them 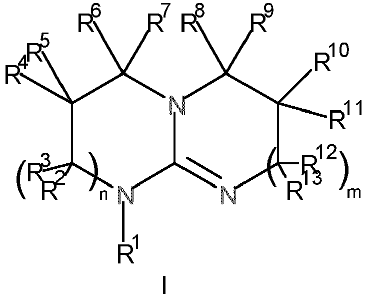
wherein R¹ to R¹³ are independently selected from the group of H and C₁ to C₄ alkyl and n and m are a natural number independently selected from the group of 0 and 1
by reacting a triamine in the present of a C₁-source and a solid material in the gas or liquid phase under inert atmosphere at a temperature of at least 90°C and a pressure of 1 to 320 bar.

The inventive process is advantageous when the solid material is selected from the group of inert materials and solid acid catalysts.

The inventive process is advantageous when the solid material is a solid acid catalyst selected from the group of aluminum silicates, aluminum oxide and zeolites.

The inventive process is advantageous, when the triamine and C₁-source are dissolved in a solvent selected from the group of methanol, ethanol, iso-propanol, butanol, diethyleneglycol, butyldiglycol, tetrahydrofuran, diglyme, proglyme, triglyme, tetraglyme, toluene, dichlorobenzene, *N,N*-dimethylformamide and *N*-methylpyrrolidone.

The inventive process is advantageous when the solvent is methanol, diethyleneglycol or butyldiglycol.

The inventive process is advantageous when the triamine is selected from the group of *N*-(3-aminopropyl)propane-1,3-diamine (DPTA), *N*-(2-aminoethyl)ethane-1,2-diamine (DETA) and *N-*(2-aminoethyl)-1,3-propanediamine.

The inventive process is advantageous when the C₁- source is selected from the group of dimethyl carbonate, urea, dimethylformamide dimethyl acetal, carbon dioxide, ethylene carbonate, propylene carbonate, phosgene and cyanamide.

The inventive process is advantageous when the process provides the following three steps:
I) reaction of the triamine in the present of the C₁-source at a temperature of at least 90°C to an urea compound of formula II 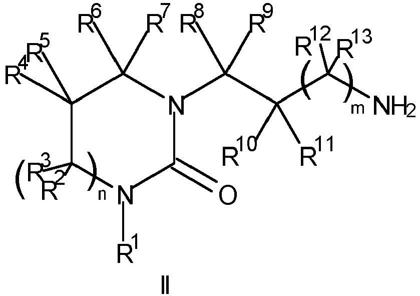 wherein R¹ to R¹³ and n and m have the same meaning as in formula I
II) dissolving the crude urea compound of formula II of step I) in a solvent and
III) cyclisation of the crude urea compound of formula II from step II) in the gas or liquid phase under inert atmosphere in the presence of the solid material at a temperature of at least 120°C and a pressure in the range of 1 to 320 bar.

The inventive process is advantageous when the process is made in the gas phase at a temperature of at least 150°C and a pressure of 1 to 35 bar.

The inventive process is advantageous when catalytical amounts of organic base or acid are present in step I of the inventive process.

The inventive process is advantageous when the solvent of step II) of the inventive process is selected from the group of methanol, ethanol, iso-propanol, butanol, diethyleneglycol, butyldiglycol, tetrahydrofuran, diglyme, proglyme, triglyme, tetraglyme, toluene, dichlorobenzene, N,N-dimethylformamide and *N*-methylpyrrolidone.

The inventive process is advantageous when a solvent selected from the group of methanol, ethanol, iso-propanol, butanol, diethyleneglycol, butyldiglycol, tetrahydrofuran, diglyme, proglyme, triglyme, tetraglyme, toluene, dichlorobenzene, *N,N*-dimethylformamide and *N-*methylpyrrolidone is used in step I of the inventive process.

The inventive process is advantageous when step II) of the inventive process will be included in step I) if the solvents of step II) and step I) are the same.

The inventive process is advantageous when the cyclic guanidine derivatives of formula I are a mixture of 1,5,7-triazabicyclo[4.4.0]dec-5-en and 7-methyl-1,5,7-triazabicyclo[4.4.0]dec-5-en.

The inventive process is advantageous when the cyclic guanidine derivatives of formula I are a mixture of 1,2,3,5,6,7-hexyhydroimidazol[1,2-*a*]pyrimidine and 8-methyl-1,2,3,5,6,7-hexahydroimidazol[1,2-*a*]pyrimidine.

The inventive process is advantageous when the cyclic guanidine derivatives of formula I are a mixture of 2,3,5,6-tetrahydro-1*H*-imidazol[1,2-*a*]imidazole and 1-methyl-2,3,5,6-tetrahydroimidazol[1,2-*a*]imidazole.

With the inventive process cyclic guanidine derivates of formula I or mixture of them 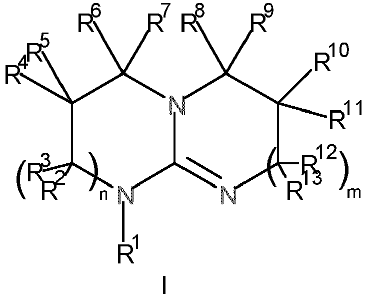 are produced. In this formula I n and m are a natural number independently selected from the group of 0 and 1, preferred are m and n the same natural number, particularly preferred are m and n 1.

R¹ to R¹³ in formula I are independently selected from the group of H and C₁ to C₄ alky groups. C₁ to C₄ alkyl group means methyl, ethyl, isopropyl, n-propyl, n-butyl, isobutyl and tert.-butyl. R¹ to R¹³ are preferred independently selected from the group of H and methyl, particularly preferred are R² to R¹³ H and R¹ is selected from the group of H and methyl.

The preferred cyclic guanidine derivatives of formula I which are available by the inventive process are selected from the group of 1,5,7-triazabicyclo[4.4.0]dec-5-en, 7-methyl-1,5,7-triazabicyclo[4.4.0]dec-5-en, 1,2,3,5,6,7-hexyhydroimidazol[1,2-*a*]pyrimidine, 8-methyl-1,2,3,5,6,7-hexahydroimidazol[1,2-*a*]pyrimidine, 2,3,5,6-tetrahydro-1*H*-imidazol[1,2-*a*]imidazole and 1-methyl-2,3,5,6-tetrahydroimidazol[1,2-*a*]imidazole.

The preferred mixtures of cyclic guanidine derivates of formula I are those that comprise 1,5,7-triazabicyclo[4.4.0]dec-5-en and 7-methyl-1,5,7-triazabicyclo[4.4.0]dec-5-en, 1,2,3,5,6,7-hexyhydroimidazol[1,2-*a*]pyrimidine and 8-methyl-1,2,3,5,6,7-hexahydroimidazol[1,2-a]pyrimidine or 2,3,5,6-tetrahydro-1*H*-imidazol[1,2-*a*]imidazole and 1-methyl-2,3,5,6-tetrahydroimidazol[1,2-*a*]imidazole.

In the inventive process triamines react with a C₁-source. The triamines that are used for the inventive process are selected from the group of *N*-(3-aminopropyl)propane-1 ,3-diamine (DPTA), *N*-(2-aminoethyl)ethane-1,2-diamine (DETA) and *N*-(2-aminoethyl)-1,3-propanediamine. The use of *N*-(3-aminopropyl)propane-1,3-diamine (DPTA) is preferred.

The C₁-source that is used in the inventive process is selected from the group of dimethyl carbonate, urea, dimethylformamide dimethyl acetal, carbon dioxide, ethylene carbonate, propylene carbonate, phosgene and cyanamide. The use of dimethyl carbonate, urea, carbon dioxide, ethylene carbonate or propylene carbonate is preferred. More preferred is the use of dimethyl carbonate, urea or carbon dioxide.

During the inventive process the C₁-source is always used in an excess which means the C₁-source is used in amounts of 1.01 to 1.50 molar equivalents, preferably 1.01 to 1.20 molar equivalents compared to the used triamine. When carbon dioxide is used as C₁-source, a large excess in the range of 10 to 500 molar equivalents of carbon dioxide compared to the used triamine is employed.

For the inventive process the starting products triamine and the C₁-source can be dissolved in a solvent or can be reacted without solvent. The reaction of the triamine with the C₁-source without solvent is preferred. If a solvent is used, the solvent will be a protic solvent selected from the group of methanol, ethanol, iso-propanol, butanol, diethyleneglycol and butyldiglycol or an aprotic solvent selected from the group of tetrahydrofuran, diglyme, proglyme, triglyme, tetraglyme, toluene, dichlorobenzene, *N,N*-dimethylformamide and *N*-methylpyrrolidone. Solvents selected from the group of methanol and butyldiglycol and tetrahydrofuran are preferred. Solvents like methanol, diethyleneglycol and butyldiglycol are particularly preferred.

The inventive process can be operated in a gas or liquid phase with a solid material. The operation in the gas phase with a solid material is preferred.

The solid material is placed in a reactor. The process of the invention is suitable in principle for pressure-resistant reactors as customarily used for reactions over heterogeneous catalysts involving feeding one gaseous and if applicable one or more liquid reactants to the reactor. These include the generally customary reactors for gas- and liquid phase reactions, for example tubular reactors, shell and tube reactors and gas circulation reactors. A specific embodiment of the tubular reactors is that of shaft reactors. Reactors of this kind are known in principle to the person skilled in the art. More particularly, a cylindrical reactor having a vertical longitudinal axis is used, having, at the base or top of the reactor, an inlet apparatus or a plurality of inlet apparatuses for feeding in a reactant mixture comprising at least one gaseous and if applicable at least one liquid component. If desired, substreams of the gaseous and/or the liquid reactant can be fed to the reactor additionally via at least one further feed apparatus.

In the preferred gas phase reaction the starting compounds like triamine, C₁-source, the inert gas and optionally the solvent are evaporated in an evaporator before reaching the solid material in the reactor. The evaporator can be an evaporator bed inside the reactor that is heated by the reactor heating and is made of inert materials selected from the group of Raschig rings, glass spheres, steel wire mesh, or wire gauze rings or it can be an extra oil or steam heated evaporator apparatus that is located outside of the reactor in front of the top of the reactor. The evaporation temperature is in the range of 80 to 280 °C, preferably in the range of 120 to 250°C. During evaporation the pressure ranges from 1 to 35, preferably from 1 to 25 bar.

For the gas phase reaction the starting compounds will get into contact with the solid material after evaporation. For the liquid phase reaction the starting compounds like triamine, C₁-source, the inert gas and optionally the solvent get directly into contact with the solid material. If the compound of formula II is dissolved in a solvent in step II of the inventive process this will happen inside or outside of the reactor before reaching the solid material. The flow direction of the gas or liquid phase through the reactor will be vertical. The flow direction can be from the top of the reactor to the bottom or vice versa. Preferred is the flow direction from the top of the reactor to the bottom.

The solid material that is used in the gas or liquid phase is selected from the group of an inert material, a solid acid catalyst or mixtures or layers of inert materials and solid acid catalysts. The inert material is selected from the group of Raschig rings, glass spheres, steel wire mesh, wire gauze rings and ceramic materials. The solid acid catalyst is selected from the group of aluminum silicates, aluminum oxide, zeolites or mixtures of these solid acids. The solid material can have different shapes and sizes. All shapes and sizes of the solid material are possible that are known to the person skilled in the art if they will allow a constant flow through the reactor. These shaped bodies are selected from the group of tablets, extrudates, split, spherical materials. Independent of material type and shape, the diameter of these shaped bodies is in the range of 1 mm to 20 mm.

The solid material is fixed inside the reactor and is the reactor bed. The reactor bed can include only inert material or inert material and one or more solid acid catalysts. A preferred reactor bed includes inert material and one or more solid acid catalysts. As part of the reactor bed inert material is at the start and at the end of the reactor bed in order to fix the position of the bed inside the reactor. If a solid acid catalyst is used, the solid acid catalyst will be located between the inert material at the start and the end of the reactor bed. The solid acid catalyst can be a single solid acid catalyst or two or more different solid acid catalysts that are mixed up or arranged in layers wherein inert material between each layer can be located. A preferred reactor bed includes inert material at the start and end of the reactor bed and a solid acid catalyst selected from the group of aluminum silicates, aluminum oxide and mixtures of them.

During the gas and or liquid phase reaction an inert gas is used which is selected from the group of N₂ and argon. With the inert gas the space velocity of the reaction can be controlled. Normally the space velocity is in the range of 50 to 10000 l/h.

The temperature of the reaction is at least 90 °C, preferred 150°C to 350 °C, more preferred in the range of 220°C to 300°C. The pressure during the inventive process is in the range between 1 and 100 bar. During the gas phase reaction of the inventive process the pressure is preferred to be in the range of 1 to 35 bar. During the liquid phase reaction of the inventive process the preferred pressure is equal to or below 100 bar.

The inventive process can be a single step, a two- or a three-step process. In the single step process the triamine, the C1-source, the inert gas and optionally the solvent will react in the gas or liquid phase directly to one of the compounds of formula I or to a mixture of compounds of formula I. In a two-step process the solvent of step I will be the same as that of step II. In this case step II is included in step I and the urea compound of formula II can be introduced directly in step III of the cyclization. The inventive process will be operated in three steps if in step I no solvent is used or if in step II another solvent than in step I is used. The single and three-step process of the inventive process is preferred. The single step process is particularly preferred.

In another embodiment of the invention the reaction can be separated in more than one step. Three steps are preferred. In the first step the triamine is reacted with the C₁-scource to the corresponding urea compound of formula II 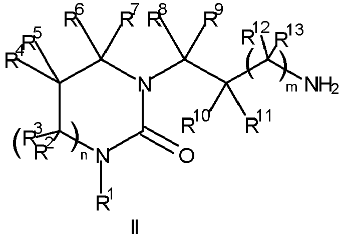 wherein R¹ - R¹³, m and n have the same meaning as in formula I. In the reaction of triamine and C₁-source in step I of the inventive process catalytic amounts of an organic base or organic acid can be used. Catalytic amounts are amounts in the range of 0.01 to 10 mol.-%, preferably 0.1 to 5.mol-% of the used triamine. Either an organic base or an organic acid can be used in step I of the inventive process. The organic base is selected from the group of strong organic bases with a pkₐ value of at least 15. The organic acid is selected from the group of sulfonic or carboxylic acids. Nitrogen containing organic bases, such as guanidines, are particularly preferred.

The reaction of the triamine and the C₁-scource can be conducted in the presence of a solvent or without a solvent. The reaction without solvent is preferred. If a solvent is used it will be a protic or an aprotic solvent. The protic solvent for step I is selected from the group of methanol, ethanol, iso-propanol, butanol, diethyleneglycol and butyldiglycol. The aprotic solvent for step I is selected from the group of tetrahydrofuran, diglyme, proglyme, triglyme, tetraglyme, toluene, dichlorobenzene, *N,N*-dimethylformamide and *N*-methylpyrrolidone. The solvent is preferably selected from the group of methanol, diethyleneglycol, butyldiglycol and tetrahydrofuran. Methanol or butyldiglycol are particularly preferred as solvent.

The reaction temperature for the reaction of step I is at least 90°C. The pressure in step I will be atmospheric pressure if the C₁-source is not a gas. If the C₁-scource is CO₂ the pressure will be in the range of 20 to 100 bar in step I of the inventive process.

After step I of the inventive process a crude urea compound of formula II will be available. "Crude" means that in the mixture small amounts of byproducts are contained. These byproducts are selected from the group of starting products, like the triamines, the C₁-scource, alkylated cyclic urea derivatives as well as minor amounts of compound of formula I where R¹ is H. Minor amounts are those amounts that are smaller compared to the amounts of compounds of formula I where R¹ is H at the end of the inventive process.

The small amounts of byproducts are in the range of 0.01 to 10 wt.-% according to the urea compound of formula II. The resulting crude urea compound of formula II will be dissolved in a solvent. The solvent can be a protic or an aprotic one. It is selected from the group of methanol, ethanol, iso-propanol, butanol, diethyleneglycol and butyldiglycol, tetrahydrofuran, diglyme, proglyme, triglyme, tetraglyme, toluene, dichlorobenzene, *N,N*-dimethylformamide and *N-*methylpyrrolidone. The solvent is preferably selected from the group of methanol, diethyleneglycol and butyldiglycol and tetrahydrofuran. The particularly preferred solvents are methanol, diethyleneglycol and butyldiglycol. If in step I a solvent is used the preferred solvent of step II will be the same of step I.

Afterwards, the crude urea compound of formula II will be fed together with the inert gas and the solvent into the liquid or gas phase of the reactor that is filled with the solid material for cyclization. If the cyclization takes place in the gas phase an evaporator will be located before the reactor bed. This can be done either at the top of the reactor or inside the reactor before the reactor bed. In the evaporator the crude urea and the solvent are evaporated together before this combined gas is fed together with the inert gas to the reactor bed including the solid material. If the cyclization takes place in the liquid phase the crude urea compound of formula II will first be dissolved in the solvent and then fed to the top or bottom of the reactor together with the inert gas.

The temperature of the cyclization in step III of the inventive process is at least 120°C, preferably at least 150°C, more preferably in the range of 150 to 300°C. The pressure during step III is in the range of 1 to 320 bar, preferably 1 to 200 bar.

The preferred step III is made in the gas phase at a temperature of at least 150°C and a pressure of 1 to 35 bar.

Often mixtures of the cyclic guanidine derivates of formula I of the inventive process are obtained. The preferred mixtures comprise 1,5,7-triazabicyclo[4.4.0]dec-5-en and 7-methyl-1,5,7-triazabicyclo[4.4.0]dec-5-en, 1,2,3,5,6,7-hexyhydroimidazol[1,2-*a*]pyrimidine and 8-methyl-1,2,3,5,6,7-hexahydroimidazol[1,2-*a*]pyrimidine or 2,3,5,6-tetrahydro-1*H*-imidazol[1,2-a]imidazole and 1-methyl-2,3,5,6-tetrahydroimidazol[1,2-*a*]imidazole. The particular preferred mixture is a mixture of 1,5,7-triazabicyclo[4.4.0]dec-5-en and 7-methyl-1,5,7-triazabicyclo[4.4.0]dec-5-en.

### Examples:

For the further examples the following abbreviation will have the following meaning:
DPTA: dipropylenetriamine (3,3'-diaminodipropylamine)
DPTU: 1-(3-aminopropyl)tetrahydropyrimidin-2(1H)-one
TBD: 1,5,7-triazabicyclo[4.4.0]dec-5-en
Me-TBD: 7-methyl-1,5,7-triazabicyclo[4.4.0]dec-5-en

### Reaction of DPTA with CO₂:

### Example 1:

40 mL of a 50 wt% solution of DPTA in methanol was transferred to a 0.3 L high-pressure laboratory autoclave equipped with an overhead stirrer and a gas inlet. At room temperature, the autoclave was purged three times with carbon dioxide (5 bar). The pressure was then again increased to 5 bar and the stirrer was started. Within 20 min, carbon dioxide was injected in portions to establish a pressure of 8 to 10 bar. Then, the autoclave was heated to 180 °C. Upon reaching 180 °C, the pressure was increased to 61 bar by injecting further carbon dioxide. Upon reaching 61 bar, the reaction mixture was stirred for 16 hours. Thereafter, the reaction mixture was cooled to room temperature and the autoclave was depressurized to ambient pressure. The reaction mixture was analyzed by gas chromatography.

The composition of the reaction mixture (without solvents) is given in area percent and was as follows:
11% DPTU
6% TBD
20% 1-methyltetrahydropyrimidin-2(1H)-one
27% 1-ethyltetrahydropyrimidin-2(1H)-one

### Example 2:

40 mL DPTA was transferred to a 0.3 L high-pressure laboratory autoclave equipped with an overhead stirrer and a gas inlet. At room temperature, the autoclave was purged three times with carbon dioxide (3 bar). The pressure was then again increased to 5 bar and the stirrer was started. Within 20 min, carbon dioxide was injected in portions to establish a pressure of 20 bar. Then, the autoclave was heated to 180 °C. Upon reaching 180 °C, the pressure was increased to 61 bar by injecting further carbon dioxide. Upon reaching 62 bar, the reaction mixture was stirred for 16 hours. Thereafter, the reaction mixture was cooled to room temperature and the autoclave was depressurized to ambient pressure. The reaction mixture was analyzed by gas chromatography.

The composition of the reaction mixture (without solvents) is given in area percent and was as follows:
31% DPTU
6% TBD
16% 1-methyltetrahydropyrimidin-2(1H)-one
22% 1-ethyltetrahydropyrimidin-2(1H)-one

### Example 3:

40 mL of a 50 wt% solution of DPTA in N-methylpyrrolidone was transferred to a 0.3 L high-pressure laboratory autoclave equipped with an overhead stirrer and a gas inlet. At room temperature, the autoclave was purged three times with carbon dioxide (3 bar). The pressure was then again increased to 5 bar and the stirrer was started. Within 20 min, carbon dioxide was injected in portions to establish a pressure of 15 bar. Then, the autoclave was heated to 180 °C. Upon reaching 180 °C, the pressure was increased to 61 bar by injecting further carbon dioxide. Upon reaching 64 bar, the reaction mixture was stirred for 16 hours. Thereafter, the reaction mixture was cooled to room temperature and the autoclave was depressurized to ambient pressure. The reaction mixture was analyzed by gas chromatography.

The composition of the reaction mixture (without solvents) is given in area percent and was as follows:
31% DPTU
5% TBD
13% 1-methyltetrahydropyrimidin-2(1H)-one
20% 1-ethyltetrahydropyrimidin-2(1H)-one

### Reaction of DPTA with dimethyl carbonate

### Example 4:

A 1000 mL three-necked flask equipped with a reflux condenser was charged with 100 g (0.76 mol) DPTA and 6.9 g (0.05 mol) TBD. The mixture was cooled to 8 °C by using an ice bath. 75 g (0.83 mol, 1.09 equiv) Dimethyl carbonate were added dropwise after 2 h. The reaction mixture was then allowed to warm to 20 °C. After reaching 20 °C, the mixture was heated to 90 °C with an oil bath and was kept at this temperature for 6 h. The reflux condenser was then substituted by a distillation bridge and the generated methanol was distilled off under atmospheric pressure with an oil bath temperature of up to 130 °C. After distillation, the solution was cooled to 20 °C and was used for the next step(s) as received and without further purification.

### Exemplary product mixture composition:

The composition of the reaction mixture (without solvents) is given in area percent and was as follows
78% DPTU
11% DPTA
6% TBD

### Step II and III:

### Example 5:

Gas phase cyclization of 1-(3-aminopropyl)tetrahydropyrimidin-2(1H)-one (DPTU) to 1,5,7-triazabicyclo[4.4.0]dec-5-en (TBD)

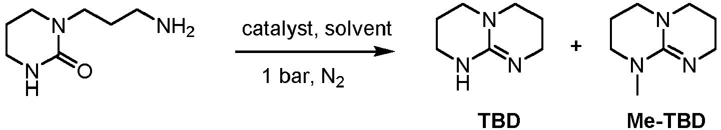

A double-walled glass reactor of 1000 mm length, diameter 40 mm with oil heating, a quartz frit at the bottom, an inlet for liquid and gaseous feeds at the top connected to a pump and gaseous feeds (N₂) measured via rotameters, was set up vertically and the outlet (bottom) was connected to a collecting flask. The off-gas was connected to a laboratory hood vent. Into the center of the reactor, a glass tube was put from the top down and a flexible thermocouple was introduced into this tube. The reactor was filled in three layers. First, 200 mL of Raschig rings composed of steel wire mesh (diameter 5 mm) were loaded onto the quartz frit. Then a catalyst (100 mL, 3 mm split/excrudates) was introduced. The height of the catalyst bed was about 80 mm. Above the catalyst bed, 700 mL of Raschig rings were loaded that served as an evaporator and heating zone for the liquid feed and the gas feed.

The tubular reactor was heated up to the reaction temperature (253 °C).

DPTU was used as a crude solution (85 area% DPTU, 10 area% DPTA, 2 area% TBD) from the reaction of dimethyl carbonate with DPTA with catalytic amounts of TBD as disclosed in example 4.

The crude DPTU, which was obtained as described in example 4, was dissolved in a solvent (10-25 wt.-% crude DPTU) and the resulting solution was directly fed on top of the evaporator bed in the reactor. Nitrogen was fed into the reactor from the top of the evaporator bed downwards through the reactor at 1 bar. The DPTU solution evaporated through the combined action of the heating and constant entrainment by nitrogen and the evaporator bed served as a heater for the DPTU/nitrogen stream.

The combined gas stream of DPTU, solvent and nitrogen passed over the catalyst bed and was condensed in the collecting flask. Liquid samples were withdrawn regularly from the flask.

The composition of the liquid sample was determined by gas chromatography yielding the area percentage of the major components TBD and Me-TBD.

**Table 1:**

| Run No | Feed (wt.-%) | DPTU in feed (%), solvent excluded | Catalyst | Temperature (°C) | solvent | Molar ratio N₂/DPTU | Catalyst Load (kg/L/h) | TBD (%) | Me-TBD (%) | DPTU (%) |
|---|---|---|---|---|---|---|---|---|---|---|
| 1 | 25 | 85 | A | 253 | MeOH | 60 | 0.05 | 60 | 8 | 8 |
| 2 | 25 | 85 | A | 253 | MeOH | 92 | 0.03 | 63 | 10 | 5 |
| 3 | 50 | 85 | A | 253 | MeOH | 46 | 0.06 | 63 | 4 | 13 |
| 4 | 25 | 85 | A | 253 | THF | 55 | 0.05 | 69 | 7 | 2 |
| 5 | 25 | 85 | B | 253 | MeOH | 59 | 0.03 | 60 | 10 | 5 |
| 6 | 25 | 85 | B | 253 | MeOH | 92 | 0.05 | 63 | 10 | 3 |

| | | | | | | | | | | |
|---|---|---|---|---|---|---|---|---|---|---|
| Catalyst A is a silica-doped aluminum oxide, 3 mm split Catalyst B an amorph aluminum oxide, 3 mm extrudates | | | | | | | | | | |

### Example 6:

A steel reactor of 770 mm length, wall thickness 3 mm and inner diameter 12 mm with electric heating was used. The reactor was filled in three layers. First, the reactor was filled with 3-5 wire gauze rings (3 mm) and 5 mL glass spheres, then with the catalyst A of example 5 (90 mL, 3 mm split) or steatite rings (70 mL, 4x3.5x2 mm) and then again with 5 mL glass spheres and 3-5 wire gauze rings (3 mm). The solution of the starting material comprising crude DPTU and the solvent was added via pump and was evaporated using an oil heated evaporator at 255 °C. Nitrogen gas was introduced into the plant in front of the evaporator. The outlet tubing of the reactor was heated to 70 to 100 °C and was connected to a collecting container. Another feed (typically solvent) could be added after the outlet of the reactor to dilute the product feed.

The reactor was heated to reaction temperature (up to 350 °C).

The crude DPTU, which was obtained as described in example 4, was dissolved in a solvent (20-25 wt.-%) and directly fed into the evaporator of the plant. The combined gas stream of DPTU, solvent and nitrogen passed over the catalyst bed, additional solvent was added after the reactor and the product mixture was condensed in the collecting flask. Liquid samples were withdrawn regularly from the flask.

The composition of the liquid sample was determined by gas chromatography yielding the area percentage of the major components.

**Table 2: Results with catalyst A.**

| Run No | Feed (wt.-%) | DPTU in feed (%), solvent excluded | Pressure (bar) | solvent | Temperature reactor (°C) | Molar ratio N₂/DPTU | Catalyst Load (kg/L/h) | TBD (%) | Me-TBD (%) | DPTU (%) |
|---|---|---|---|---|---|---|---|---|---|---|
| 1 | 25 | 72 | 20 | MeOH | 260 | 50 | 0.05 | 0 | 21 | 6 |
| 2 | 25 | 76 | 10 | MeOH | 280 | 50 | 0.05 | 4 | 37 | 3 |
| 3 | 20 | 72 | 1 | Butyldiglycol | 270 | 50 | 0.05 | 33 | 0 | 34 |
| 4 | 20 | 72 | 1 | Butyldiglycol | 275 | 50 | 0.03 | 41 | 0 | 19 |

The results of table 2 as compared to table 1 show that the formation of compounds of formula I can be controlled by the pressure of the reaction. A higher pressure results in the formation of alkylated guanidines.

**Table 3: Results with steatite rings (4x3.5x2 mm)**

| Run No | Feed (wt.-%) | DPTU in feed (%), solvent excluded | Pressure (bar) | solvent | Temperature reactor (°C) | Molar ratio N₂/DPTU | Inert Material Load (kg/L/h) | TBD (%) | Me-TBD (%) | DPTU (%) |
|---|---|---|---|---|---|---|---|---|---|---|
| 1 | 25 | 76 | 10 | MeOH | 280 | 50 | 0.05 | 29 | 10 | 20 |
| 2 | 25 | 76 | 10 | MeOH | 285 | 50 | 0.03 | 20 | 30 | 5 |

## Claims

1. A Process for the production of cyclic guanidine derivates of formula I or mixtures of them 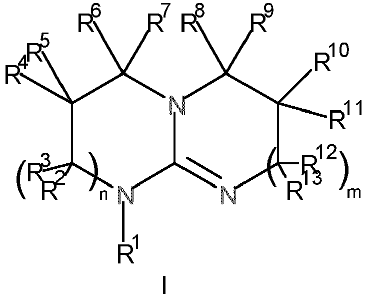 wherein R¹ to R¹³ are independently selected from the group of H and C₁- to C₄ alkyl and n and m are a natural number independently selected from the group of 0 and 1 by reacting a triamine in the present of a C₁-source in a reactor filled with a solid material selected from the group of inert material, aluminium silicates, aluminium oxide, zeolites or mixtures or layers of these in the gas or liquid phase under inert atmosphere at a temperature of at least 90°C and a pressure of 1 to 320 bar.

2. The process according to claim 1, wherein the triamine and C₁-source are solved in a solvent selected from the group of methanol, ethanol, iso-propanol, butanol, diethyleneglycol, butyldiglycol, tetrahydrofuran, diglyme, proglyme, triglyme, tetraglyme, toluene, dichlorobenzene, *N,N*-dimethylformamide and *N*-methylpyrrolidone.

3. The process according to one of claim 1 to 2, wherein the solvent is methanol, diethyleneglycol or butyldiglycol.

4. The process according to one of the claim 1 to 3, wherein the triamine is selected from the group of *N*-(3-aminopropyl)propane-1 ,3-diamine (DPTA), *N*-(2-aminoethyl)ethane-1,2-diamine (DETA) and *N*-(2-aminoethyl)-1,3-propanediamine.

5. The process according to one of the claim 1 to 4, wherein the C₁- source is selected from the group of dimethyl carbonate, urea, dimethylformamide dimethyl acetal, carbon dioxide, ethylene carbonate, propylene carbonate, phosgene and cyanamide.

6. The process according to one of claim 1 to 6 wherein the process provides the following three steps:
I) reaction of the triamine in the present of the C₁-source at a temperature of at least 90°C to an urea compound of formula II 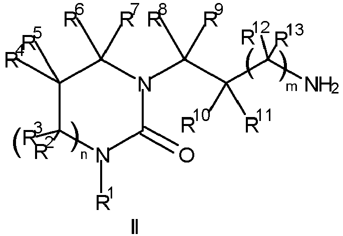 wherein R¹ to R¹³ and n and m have the same meaning as in formula I
II) dissolving the crude urea compound of formula II of step I) in a solvent
III) cyclisation of the crude and solved urea compound of formula II from step II) in the gas or liquid phase under inert atmosphere in the present of the solid material at a temperature of at least 120°C and a pressure in the range of 1 to 320 bar.

7. The process according to claim 1 to 6, wherein the process is made in the gas phase at a temperature of at least 150°C and a pressure of 1 to 35 bar.

8. The process according to one of claim 6 or 7, wherein in step I) of the process catalytical amounts of an organic base or acid are present.

9. The process according to one of claim 6 to 8, wherein the solvent of step II) of the process is selected from the group of methanol, ethanol, iso-propanol, butanol, diethyleneglycol, butyldiglycol, tetrahydrofuran, diglyme, proglyme, triglyme, tetraglyme, toluene, dichlorobenzene, *N,N*-dimethylformamide and *N*-methylpyrrolidone.

10. The process according to one of claim 6 to 9, wherein in step I) of the process a solvent selected from the group of methanol, ethanol, iso-propanol, butanol, diethyleneglycol, butyldiglycol, tetrahydrofuran, diglyme, proglyme, triglyme, tetraglyme, toluene, dichlorobenzene, *N,N*-dimethylformamide and *N*-methylpyrrolidone is used.

11. The process according to one of claim 6 to 10, wherein step II) is included in step I) when the solvents of step II) and step I) are the same.

12. The process according to one of claim 1 to 11, wherein cyclic guanidine derivates of formula I are a mixture of 1,5,7-triazabicyclo[4.4.0]dec-5-en and 7-methyl-1,5,7-triazabicyclo[4.4.0]dec-5-en.

13. The process according to one of claim 1 to 11, wherein cyclic guanidine derivates of formula I are a mixture of 1,2,3,5,6,7-hexyhydroimidazol[1,2-*a*]pyrimidine and 8-methyl-1,2,3,5,6,7-hexahydroimidazol[1,2-*a*]pyrimidine.

14. The process according to one of claim 1 to 11, wherein cyclic guanidine derivates of formula I are a mixture of 2,3,5,6-tetrahydro-1*H*-imidazol[1,2-*a*]imidazole and 1-methyl-2,3,5,6-tetrahydroimidazol[1,2-*a*]imidazole.

## Patentansprüche

1. Verfahren zur Herstellung von cyclischen Guanidinderivaten der Formel I oder Gemischen davon 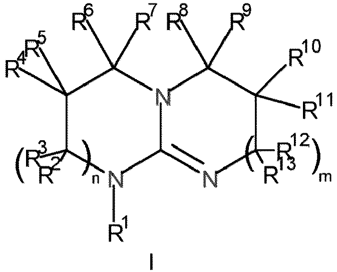 wobei R¹ bis R¹³ unabhängig aus der Gruppe von H und C₁- bis C₄-Alkyl ausgewählt sind und n und m für eine ganze Zahl, die unabhängig aus der Gruppe von 0 und 1 ausgewählt ist, stehen, durch Umsetzen eines Triamins in Gegenwart einer C₁-Quelle in einem Reaktor, der mit einem aus der Gruppe inertes Material, Aluminiumsilikate, Aluminiumoxid, Zeolithe oder Gemische oder Schichten davon ausgewählten festen Material gefüllt ist, in der Gas- oder Flüssigphase unter Inertatmosphäre bei einer Temperatur von mindestens 90 °C und einem Druck von 1 bis 320 bar.

2. Verfahren nach Anspruch 1, wobei das Triamin und die C₁-Quelle in einem Lösungsmittel aus der Gruppe Methanol, Ethanol, Isopropanol, Butanol, Diethylenglykol, Butyldiglykol, Tetrahydrofuran, Diglyme, Proglyme, Triglyme, Tetraglyme, Toluol, Dichlorbenzol, *N,N-*Dimethylformamid und *N*-Methylpyrrolidon gelöst werden.

3. Verfahren nach Anspruch 1 oder 2, wobei es sich bei dem Lösungsmittel um Methanol, Diethylenglykol oder Butyldiglykol handelt.

4. Verfahren nach einem der Ansprüche 1 bis 3, wobei das Triamin aus der Gruppe *N*-(3-Aminopropyl)propan-1,3-diamin (DPTA), *N*-(2-Aminoethyl)ethan-1,2-diamin (DETA) und *N*-(2-Aminoethyl)-1,3-propandiamin ausgewählt wird.

5. Verfahren nach einem der Ansprüche 1 bis 4, wobei die C₁-Quelle aus der Gruppe Dimethylcarbonat, Harnstoff, Dimethylformamiddimethylacetal, Kohlendioxid, Ethylencarbonat, Propylencarbonat, Phosgen und Cyanamid ausgewählt wird.

6. Verfahren nach einem der Ansprüche 1 bis 6, wobei das Verfahren die folgenden drei Schritte umfasst:
I) Umsetzung des Triamins in Gegenwart der C₁-Quelle bei einer Temperatur von mindestens 90 °C zu einer Harnstoffverbindung der Formel II 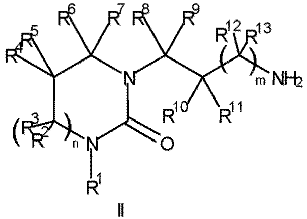 wobei R¹ bis R¹³ und n und m die gleiche Bedeutung wie in Formel I haben,
II) Lösen der rohen Harnstoffverbindung der Formel II aus Schritt I) in einem Lösungsmittel,
III) Cyclisierung der rohen und gelösten Harnstoffverbindung der Formel II aus Schritt II) in der Gas- oder Flüssigphase unter Inertatmosphäre in Gegenwart des festen Materials bei einer Temperatur von mindestens 120 °C und einem Druck im Bereich von 1 bis 320 bar.

7. Verfahren nach Anspruch 1 bis 6, wobei das Verfahren in der Gasphase bei einer Temperatur von mindestens 150 °C und einem Druck von 1 bis 35 bar durchgeführt wird.

8. Verfahren nach Anspruch 6 oder 7, wobei in Schritt I) des Verfahrens katalytische Mengen einer organischen Base oder Säure vorliegen.

9. Verfahren nach einem der Ansprüche 6 bis 8, wobei das Lösungsmittel von Schritt II) des Verfahrens aus der Gruppe Methanol, Ethanol, Isopropanol, Butanol, Diethylenglykol, Butyldiglykol, Tetrahydrofuran, Diglyme, Proglyme, Triglyme, Tetraglyme, Toluol, Dichlorbenzol, *N,N*-Dimethylformamid und N-Methylpyrrolidon ausgewählt wird.

10. Verfahren nach einem der Ansprüche 6 bis 9, wobei in Schritt I) des Verfahrens ein Lösungsmittel aus der Gruppe Methanol, Ethanol, Isopropanol, Butanol, Diethylenglykol, Butyldiglykol, Tetrahydrofuran, Diglyme, Proglyme, Triglyme, Tetraglyme, Toluol, Dichlorbenzol, *N,N*-Dimethylformamid und *N*-Methylpyrrolidon verwendet wird.

11. Verfahren nach einem der Ansprüche 6 bis 10, wobei Schritt II) in Schritt I) enthalten ist, wenn die Lösungsmittel von Schritt II) und Schritt I) gleich sind.

12. Verfahren nach einem der Ansprüche 1 bis 11, wobei es sich bei den cyclischen Guanidinderivaten der Formel I um ein Gemisch von 1,5,7-Triazabicyclo[4.4.0]dec-5-en und 7-Methyl-1,5,7-triazabicyclo[4.4.0]dec-5-en handelt.

13. Verfahren nach einem der Ansprüche 1 bis 11, wobei es sich bei den cyclischen Guanidinderivaten der Formel I um ein Gemisch von 1,2,3,5,6,7-Hexahydroimidazol[1,2-a]pyrimidin und 8-Methyl-1,2,3,5,6,7-hexahydroimidazol[1,2-*a*]pyrimidin handelt.

14. Verfahren nach einem der Ansprüche 1 bis 11, wobei es sich bei den cyclischen Guanidinderivaten der Formel I um ein Gemisch von 2,3,5,6-Tetrahydro-1*H*-imidazol[1,2-*a*]imidazol und 1-Methyl-2,3,5,6-tetrahydroimidazol[1,2-*a*]imidazol handelt.

## Revendications

1. Procédé pour la production de dérivés de guanidine cycliques de formule I ou de mélanges de ceux-ci 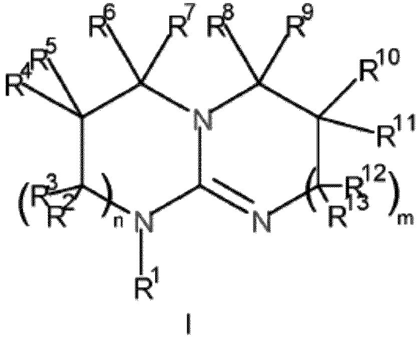
R¹ à R¹³ étant indépendamment choisis dans le groupe composé de H et alkyle en C₁₋₄ et n et m étant un nombre naturel indépendamment choisi dans le groupe de 0 et 1
par mise en réaction d'une triamine en la présence d'une source de C₁ dans un réacteur rempli avec une matière solide choisie dans le groupe d'une matière inerte, de silicates d'aluminium, d'oxyde d'aluminium, de zéolithes ou de mélanges ou de couches de ceux-ci dans la phase gazeuse ou liquide sous une atmosphère inerte à une température d'au moins 90 °C et une pression de 1 à 320 bars.

2. Procédé selon la revendication 1, la triamine et la source de C₁ étant dissoutes dans un solvant choisi dans le groupe composé par le méthanol, l'éthanol, l'iso-propanol, le butanol, le diéthylèneglycol, le butyldiglycol, le tétrahydrofuranne, le diglyme, le proglyme, le triglyme, le tétraglyme, le toluène, un dichlorobenzène, le *N,N*-diméthylformamide et la *N*-méthylpyrrolidone.

3. Procédé selon l'une des revendications 1 à 2, le solvant étant le méthanol, le diéthylèneglycol ou le butyldiglycol.

4. Procédé selon l'une des revendications 1 à 3, la triamine étant choisie dans le groupe composé de la *N*-(3-aminopropyl)propane-1,3-diamine (DPTA), la *N-*(2-aminoéthyl)éthane-1,2-diamine (DETA) et la *N-(2-*aminoéthyl)-1,3-propanediamine.

5. Procédé selon l'une des revendications 1 à 4, la source de C₁ étant choisie dans le groupe composé par le carbonate de diméthyle, l'urée, l'acétal de diméthyle du diméthylformamide, le dioxyde de carbone, le carbonate d'éthylène, le carbonate de propylène, le phosgène et le cyanamide.

6. Procédé selon l'une des revendications 1 à 6, le procédé fournissant les trois étapes suivantes :
I) réaction de la triamine en la présence de la source de C₁ à une température d'au moins 90 °C pour donner un composé de type urée de formule II 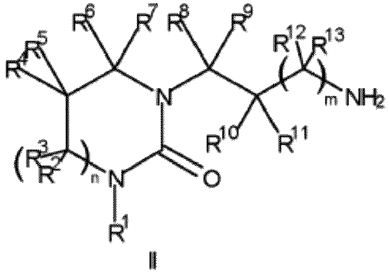 R¹ à R¹³ et n et m ayant la même signification que dans la formule I
II) dissolution du composé de type urée de formule II brut de l'étape I) dans un solvant
III) cyclisation du composé de type urée de formule II brut et dissous de l'étape II) dans la phase gazeuse ou liquide sous une atmosphère inerte en la présence de la matière solide à une température d'au moins 120 °C et une pression dans la plage de 1 à 320 bars.

7. Procédé selon les revendications 1 à 6, le procédé étant réalisé dans la phase gazeuse à une température d'au moins 150 °C et une pression de 1 à 35 bars.

8. Procédé selon l'une des revendications 6 ou 7, dans l'étape I) du procédé, des quantités catalytiques d'une base ou d'un acide organique étant présentes.

9. Procédé selon l'une des revendications 6 à 8, le solvant de l'étape II) du procédé étant choisi dans le groupe composé par le méthanol, l'éthanol, l'isopropanol, le butanol, le diéthylèneglycol, le butyldiglycol, le tétrahydrofuranne, le diglyme, le proglyme, le triglyme, le tétraglyme, le toluène, un dichlorobenzène, le *N,N*-diméthylformamide et la *N*-méthylpyrrolidone.

10. Procédé selon l'une des revendications 6 à 9, dans l'étape I) du procédé, un solvant choisi dans le groupe composé par le méthanol, l'éthanol, l'isopropanol, le butanol, le diéthylèneglycol, le butyldiglycol, le tétrahydrofuranne, le diglyme, le proglyme, le triglyme, le tétraglyme, le toluène, un dichlorobenzène, le *N,N*-diméthylformamide et la *N*-méthylpyrrolidone étant utilisé.

11. Procédé selon l'une des revendications 6 à 10, l'étape II) étant comprise dans l'étape I) lorsque les solvants de l'étape II) et de l'étape I) sont les mêmes.

12. Procédé selon l'une des revendications 1 à 11, les dérivés de guanidine cycliques de formule I étant un mélange de 1,5,7-triazabicyclo[4.4.0]déc-5-ène et de 7-méthyl-1,5,7-triazabicyclo[4.4.0]déc-5-ène.

13. Procédé selon l'une des revendications 1 à 11, les dérivés de guanidine cycliques de formule I étant un mélange de 1,2,3,5,6,7-hexyhydroimidazol[1,2-a]pyrimidine et de 8-méthyl-1,2,3,5,6,7-hexahydroimidazol[1,2-a]pyrimidine.

14. Procédé selon l'une des revendications 1 à 11, les dérivés de guanidine cycliques de formule I étant un mélange de 2,3,5,6-tétrahydro-1H-imidazol[1,2-a]imidazole et de 1-méthyl-2,3,5,6-tétrahydroimidazol[1,2-a]imidazole.
